(19) Europäisches Patentamt
European Patent Office
Office européen des brevets

(11) Publication number: **0 147 915**
**B1**

(12) **EUROPEAN PATENT SPECIFICATION**

(45) Date of publication of patent specification: **06.05.87**

(51) Int. Cl.$^4$: **C 07 C 143/22,** A 61 K 31/185

(21) Application number: **84305775.3**

(22) Date of filing: **23.08.84**

(54) **New azulene derivatives useful as anti-ulcerative and anti-flammatory agents.**

(30) Priority: **24.08.83 JP 153187/84**
**28.01.84 JP 14028/84**

(43) Date of publication of application:
**10.07.85 Bulletin 85/28**

(45) Publication of the grant of the patent:
**06.05.87 Bulletin 87/19**

(84) Designated Contracting States:
**AT BE CH DE FR GB IT LI LU NL SE**

(56) References cited:
**DE-B-1 227 896**
**US-A-3 816 612**

**ARZNEIMITTEL-FORSCHUNG 22, 1972, no. 6, *Editio Cantor* KG, Aulendorf/Württ. K. THIEMER et al.: "Biochemische Untersuchungen von Kamillen-Inhaltsstoffen"**

(73) Proprietor: **Kotobuki Seiyaku Co., Ltd.**
**6351 Ohaza Sakaki Sakaki-machi**
***Hanishina-gun Nagano-ken* (JP)**

(72) Inventor: **Takase, Kahei**
**15-12 Matsugaoka**
**Sendai-shi Miyagi-ken (JP)**
Inventor: **Yasunami, Masafumi**
**14-1-13 sanjo-machi**
**Sendai-shi Miyagi-ken (JP)**
Inventor: **Tomiyama, Tsuyoshi**
**1113 Ohaza Sakaki Sakaki-machi**
**Hanishina-gun Nagano-ken (JP)**
Inventor: **Tomiyama, Akira**
**78 Kurohiko Togura-machi**
**Hanishina-gun Nagano-ken (JP)**
Inventor: **Yanagisawa, Takashi**
**6351 Ohaza Sakaki Sakaki-machi**
**Hanishina-gun Nagano-ken (JP)**

(74) Representative: **Seaborn, George Stephen et al**
**c/o Edward Evans & Co. Chancery House 53-64**
***Chancery Lane***
**London WC2A 1SD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 147 915 B1

**Description**

Field of the Invention

This invention relates to new azulene derivatives having valuable therapeutic properties and methods of synthesizing them.

Background of the Invention

Azulene derivatives have been well known as having anti-gastric ulcer, anti-gastritis and anti-inflammation activities. However, because of their instability to light and heat, an improved stable and potent derivative has been long desired.

It will be understood from the description below that the present invention provides novel compounds having advantageous pharmaceutical properties, pharmaceutical compositions useful as anti-peptic ulcerative agents, pharmaceutical compositions useful as anti-inflammatory agents and new sodium azulene derivatives sulphonates and a method for the manufacture thereof.

Summary of the Invention

According to the invention, there are provided new compounds of the general formula (I)

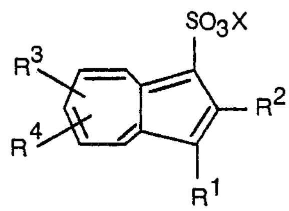

(I)

where: $R^1$ is a lower alkyl group, a group of the general formula

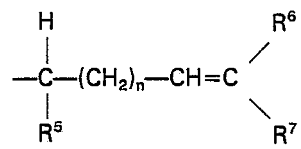

or a benzyl group, $R^5$, $R^6$ and $R^7$ each representing H or a lower alkyl group and n being 1 or 2;

$R^2$ and $R^3$ each representing H or a lower alkyl group;

$R^4$ is H or an alkoxy group; and

X is Na or $Al(OH)_2$.

Compounds of the Formula I possess potent anti-gastric ulcer and anti-inflammatory activities and therefore, they are regarded as therapeutically useful. The compounds of this invention are more potent and chemically stable than sodium guaiazulene-3-sulfonate, which has heretofore been used on account of its anti-gastric ulcer and anti-inflammatory activities.

In this specification by "lower alkyl group", in relation to $R^1$, $R^2$, $R^3$, $R^4$, $R^5$, $R^6$ and $R^7$ in Formula (I), is meant a straight chain alkyl group or a branched alkyl group having from 1 to 6 carbon atoms. In compunds of the formula I desirably the positions of $R^3$ and $R^4$ are selected from the 4, 6 and 7 positions on the 7-membered ring.

The following compounds according to the invention are of particular interest.

1) Sodium 3-methyl-azulene sulfonate
2) Sodium 3-ethyl-azulene sulfonate
3) Sodium 3-ethyl-7-isopropyl-azulene sulfonate
4) Sodium 3-ethyl-6-isopropyl-azulene sulfonate
5) Sodium 3-(1'—R, S—1', 5'-dimethyl-4'-hexane) azulene sulfonate
6) Sodium 3-n-butyl-azulene sulfonte
7) Sodium 3-methyl-2-ethyl-azulene sulfonate
8) Sodium 3-(1'—S—1', 5'-dimethyl-4'-hexene)-azulene sulfonate
9) Sodium 3-(1'—R—1', 5'-dimethyl-4'-hexene)-azulene sulfonate
10) Sodium 3-(1'—S—1', 5'-dimethyl-4'-hexene)-7-isopropyl-azulene sulfonate
11) Sodium 3-(1'—R—1', 5'-dimethyl-4'-hexene)-7-isopropyl-azulene sulfonate
12) Sodium 3-propyl-azulene sulfonate
13) Sodium 3-methyl-7-isopropyl-azulene sulfonate
14) Sodium 3-n-butyl-azulene sulfonate
15) Sodium 3-n-pentyl-azulene sulfonate
16) Sodium 5-isopropyl-azulene sulfonate

17) Sodium 7-isopropyl-3-n-propyl-azulene sulfonate
18) Sodium 7-isopropyl-3-n-butyl-azulene sulfonate
19) Sodium 7-isopropyl-3-pentyl-azulene sulfonate
20) Sodium 7-isopropyl-3-(1′—R, S—1′, 5′-dimethyl-4′-hexene)-azulene sulfonate
21) Sodium 7-isopropyl-3-(1′—R—1′, 5′-dimethyl-4′-hexene)-azulene sulfonate
22) Sodium 7-isopropyl-3-(1′—S—1′, 5′-dimethyl-4′-hexene)-azulene sulfonate
23) Sodium 7-isopropyl-3-benzyl-azulene sulfonate
24) Sodium 4-methoxy-3-methyl-azulene sulfonate
25) Sodium 4-methoxy-3-ethyl-azulene sulfonate
26) Sodium 4-methoxy-3-propyl-azulene sulfonate
27) Sodium 4-methoxy-3-butyl-azulene sulfonate
28) Sodium 4-methoxy-3-pentyl-azulene sulfonate
29) Sodium 4-methozy-3-hexyl-azulene sulfonate
30) Sodium 7-isopropyl-4-methoxy-3-methyl-azulene sulfonate
31) Sodium 7-isopropyl-4-methoxy-3-ethyl-azulene sulfonate
32) Sodium 7-isopropyl-4-methoxy-3-propyl-azulene sulfonate
33) Sodium 7-isopropyl-4-methoxy-3-buytl-azulene sulfonate
34) Sodium 7-isopropyl-4-methoxy-3-pentyl-azulene sulfonate
35) 3-methyl-azulene sulfonic acid aluminium salt
36) 3-ethyl-azulene acid aluminium salt
37) 7-isopropyl-3-methyl-azulene sulfonic acid aluminium salt
38) 7-isopropyl-3-ethyl-azulene sulfonic acid aluminium salt

The above-mentioned compounds numbered from 1 to 38, are referred to hereinafter as Compound 1 to Compound 38, respectively.

The new azulene derivatives of the general Formula (I) may be obtained by sulfonation of a compound of the general Formula (II) below and converting the sulfonic acid produced to a sodium or aluminium salt

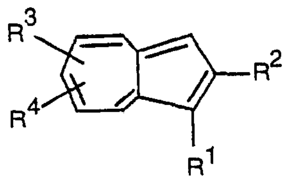

(II)

In the Formula (II), $R^1$, $R^2$, $R^3$ and $R^4$ are as defined above.

The pre-sulfonated compounds of Formula (II) may be prepared by any of several methods i) to iv) as described below.

i) Compounds of Formula (II) where $R^1$ and $R^3$ are each H or a lower alkyl and $R^2$ is H may be obtained by the method according to L. T. Scott (J. Am Chem. Soc. *102* 6311p 1980) or m. Yasunami (Chemistry Letters 579p *1980*).

ii) Where $R^1$ is

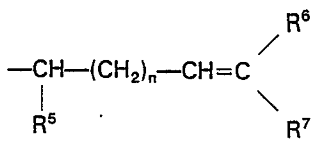

or benzyl, the compound of Formula (II) may be obtained by reacting 2H-cyclohepta(b)furan-2-one with an enamine obtained from the corresponding aldehyde of the formula:

$$R^1—CH_2CHO,$$

according to M. Yasunami (Chem. Lett. 579p 1980)

iii) Compounds of Formula (II) in which both $R^1$ and $R^2$ are lower alkyl groups may be obtained by the process (ii) above except that an enamine obtained from a ketone

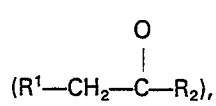,

is used, instead of aldehyde ($R^1—CH_2CH$ CHO), in reaction (ii) in the reaction, even though starting aldehyde contains a stereo specific isomer, for example, R is d- or l-citronellal and d- or l-limonene, the resulting product is found to keep its stereo specificity.

iv) Other compounds of the Formula (II) may also be prepared according to M. Yasunami (Chem. Lett. 589p 1980).

The compounds obtained as described above may be purified by distillation under reduced pressure or column chromatography and then submitted to sulfonation. Sulfonation is generally carried out in acetic anhydride by adding sulfuric acid in cooling condition, also in other cases, sulfuric anhydride-pyridine complex can be utilized. The resulting sulfonated compound is converted to its sodium salt with a solution of sodium hydroxide or sodium ethylate and then recrystallized from an alcohol.

To obtain aluminium salts, sodium sulfonates are converted by aluminium salt solutions, for example, an aqueous solution of $Al(OH)_3$, $AlCl_3$ or aluminium propoxide.

The compounds of this invention thus obtained posses improved stability to light and heat and some of these compounds have excellent anti-peptic and anti-gastric ulcer activities and are promising for therapeutic use.

For pharmaceutical purpose, the compounds of the present invention are administered to animals and human perorally, parenterally or rectally as active ingredients in customary dosage unit composition, that is, compositions in dosage unit form consisting essentially of an inert pharmaceutical carrier and one effective dosage unit of the active ingredient, such as tablets, granules, capsules, suspensions and the like. One effective dosage unit of the compounds according to the present invention is from 2 mg to 60 mg, 3 times a day for an adult.

## Examples

The following examples show the biological activity of the compounds of the invention as compared with sodium guaiazulene-3-sulfonate (GAS) as a standard, the preparation of typical compositions of the present invention and preparation of the compounds of the invention.

### Pharmacological data

Anti-peptic activity of the compounds according to the invention is tested in vitro according to V.K. Thiemer (Arznei-Forsch *22* (6), PP.1086, 1972), using bovine serum albumine as a substrate and commercially available pepsine.

Activity is expressed as % inhibition to the control value (no inhibitor added) as shown in Table 1. Also $ID_{50}$ (50% inhibiting dose) is obtained graphically.

TABLE 1 (% inhibition)

| sample / concentration (mg/ml) | Compound 3 | Compound 4 | Compound 5 | GAS |
|---|---|---|---|---|
| 0.125 | 17.304 | 17.305 | 0.260 | 14.578 |
| 0.250 | 47.596 | 28.864 | 3.570 | 50.357 |
| 0.500 | 63.570 | 65.747 | 24.480 | 58.604 |
| 1.000 | 97.142 | 55.844 | 50.356 | 75.130 |
| $ID_{50}$(mg/ml) | 0.297 | 0.510 | 1.207 | 0.358 |

$ID_{50}$: 50% inhibition doses

### Pharmacological data 2

In order to ascertain the ani-ulcerative activity in vitro, Shay's rat (Gastro enterology *26*, P.906) is selected. Rats are fasted for 48 hours and pylorus ligation is carried out under light anesthesia. The compound to be tests is given perorally. 16 hours after ligation the animals are sacrificed and the stomach is removed. The induced erosion is measured in area and scored as ulcer index (U.I.). The data are expressed as % inhibition to the control valve from the equation described below.

$$\% \text{ inhibition} = \frac{\text{Control value} - \text{test value}}{\text{control value}} \times 100$$

| test compound | dose (mg/kg) | % inhibition |
|---|---|---|
| Compound 1 | 500 mg | 52.4 |
| Compound 2 | 500 mg | 100 |
| Compound 3 | 500 mg | 100 |
| Compound 4 | 500 mg | 100 |
| Compound 5 | 500 mg | 95.5 |
| Compound 8 | 500 mg | 69.9 |
| Compound 12 | 100 mg | 78.5 |
| Compound 13 | 100 mg | 69.7 |
| Compound 14 | 100 mg | 32.7 |
| Compound 15 | 100 mg | 64.3 |
| Compound 16 | 100 mg | 62.0 |
| Compound 17 | 100 mg | 31.2 |
| Compound 18 | 100 mg | 17.2 |
| Compound 19 | 100 mg | −56.3 |
| Compound 20 | 100 mg | 43.8 |
| Compound 21 | 100 mg | 14.5 |
| Compound 22 | 100 mg | −19.5 |
| Compound 23 | 100 mg | 9.8 |
| Compound 24 | 100 mg | 63.4 |
| Compound 25 | 100 mg | 75.6 |
| Compound 26 | 100 mg | 75.6 |
| Compound 27 | 100 mg | 66.3 |
| Compound 28 | 100 mg | 42.0 |
| Compound 29 | 100 mg | 38.0 |
| Compound 30 | 100 mg | 58.7 |
| Compound 31 | 100 mg | 67.6 |
| Compound 32 | 100 mg | 44.1 |
| Compound 33 | 100 mg | 64.8 |
| Compound 34 | 100 mg | −14.6 |
| Compound 35 | 100 mg | 16.5 |
| Compound 36 | 100 mg | 28.6 |
| Compound 37 | 100 mg | 58.4 |
| Compound 38 | 100 mg | 40.3 |

Toxicity

The acute toxicity of Compound 3 is determined by the Litchfield & Wilcoxon method using SD rats. The $LD_{50}$ values of the compound is shown below.

$LD_{50}$ VALUE (mg/kg)

| Administration route | ♂ | ♀ |
|---|---|---|
| P.O. | 1000(833.3 — 1200) | 1200(983.6 — 146.4) |
| I.P. | 165(136.4 — 199.8) | 180(153.8 — 210.6) |
| I.V. | 130 | 153(111.7 — 209.6) |

(95% confidence limits)

It should be emphasized that stability of Compound 3 is greatly improved compared with that of sodium guaiazulene sulfonate which has hitherto been used clinically.

Because the instability of sodium guaiazulene sulfonate has limited its pharmaceutical utility, the improved stability of the compounds of this invention is meritorious.

The following data show the stability of Compound 3. The data are expressed in % residue of their original compounds after storage in each condition.

| storage | 40°C 96 hours | 60°C 192 hours | 70°C 240 hours | 80°C 288 hours |
|---|---|---|---|---|
| sodium guaiazulene sulfonate | 100% | 51.3% | 9.3% | 1.5% |
| compound 3 | 100% | 100% | | 100% |

The following examples illustrate typical pharmaceutical dosage unit compositions comprising a compound of the present invention as an active ingredient and represent the modes contemplated for putting the invention into practical use.

Pharmaceutical Example 1

Granules were prepared from the following ingredients

| | |
|---|---|
| Sodium 3-ethyl-7-isopropyl-azulene sulfonate | 3 mg |
| Lactose | 433 mg |
| L-glutamine | 60 mg |
| Methyl cellulose | 4 mg |
| | total 500 mg |

Preparation

In this example and the following pharmaceutical examples, the ingredients were intimately mixed with each other and the mixture was granulated in a conventional manner.

Pharmaceutical Example 2

The granules were prepared from the following ingredients

| | |
|---|---|
| Sodium 3-ethyl-7-isopropyl-azulene sulfonate | 6 mg |
| Lactose | 430 mg |
| L-glutamine | 60 mg |
| Methyl cellulose | 4 mg |
| | total 500 mg |

Pharmaceutical Example 3

Granules were prepared from the following ingredients

| | |
|---|---|
| Sodium 3-ethyl-7-isopropyl-azulene sulfonate | 9 mg |
| Lactose | 427 mg |
| L-glutamine | 60 mg |
| Methyl cellulose | 4 mg |
| | total 500 mg |

Pharmaceutical Example 4

Granules were prepared from the following ingredients

| | |
|---|---|
| Sodium 3-ethyl-7-isopropyl-azulene sulfonate | 3 mg |
| Lactose | 393 mg |
| Synthetic hydrotalcite | 100 mg |
| Methyl cellulose | 4 mg |
| | total 500 mg |

Pharmaceutical Example 5

Granules were prepared from the following ingredients

| | |
|---|---|
| Sodium 3-ethyl-7-isopropyl-azulene sulfonate | 6 mg |
| Lactose | 390 mg |
| Synthetic hydrotalcite | 100 mg |
| Methyl cellulose | 4 mg |
| | total 500 mg |

Pharmaceutical Example 6

Granules were prepared from the following ingredients

| | |
|---|---|
| Sodium 3-ethyl-7-isopropyl-azulene sulfonate | 9 mg |
| Lactose | 387 mg |
| Synthetic hydrotalcite | 100 mg |
| Methyl cellulose | 4 mg |
| | total 500 mg |

Pharmaceutical Example 7

Granules were prepared from the following ingredients

| | |
|---|---|
| Sodium 3-ethyl-7-isopropyl-azulene sulfonate | 3 mg |
| Lactose | 150 mg |
| Starch | 343 mg |
| Methyl cellulose | 4 mg |
| | total 500 mg |

Pharmaceutical Example 8

Granules were prepared from the following ingredients

| | |
|---|---|
| Sodium 3-ethyl-7-isopropyl-azulene sulfonate | 6 mg |
| Lactose | 150 mg |
| Starch | 340 mg |
| Methyl cellulose | 4 mg |
| | total 500 mg |

Pharmaceutical Example 9

Granules were prepared from the following ingredients

| | |
|---|---|
| Sodium 3-ethyl-7-isopropyl-azulene sulfonate | 9 mg |
| Lactose | 150 mg |
| Starch | 337 mg |
| Methyl cellulose | 4 mg |
| | total 500 mg |

Any one of the other compounds of Formula I may be substituted for the particular active ingredient in the above pharmaceutical Examples 1 to 9. Likewise, the amount of active ingredient in these illustrative pharmaceutical examples may be varied to achieve the dosage unit range set forth above, and the amounts and nature of the inert pharmaceutical carrier ingredients may be varied to meet particular requirements.

The following examples illustrate the preparation of compounds according to the invention.

Example 1

Sodium 1-methyl-azulene-3-sulfonate

The starting compound, 1-methyl azulene, may be prepared as illustrated below according to Yasunami et al (Chemistry Letters, pp579—582, 1980).

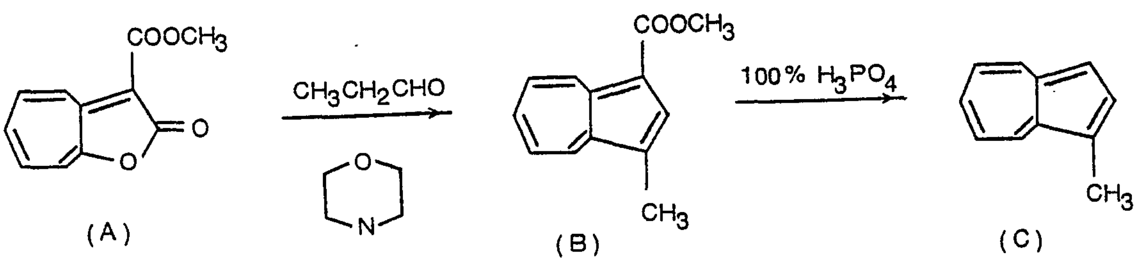


(A) (B) (C)

(Step 1) 1-methyl-3-carboxymethyl azulene (B).

A solution of oxazulene (A) 6 g, propyl aldehyde 5.22 g and morpholine 7.84 g in 120 mg of EtOH was refluxed for 4 hours. The mixture was cooled and the organic solvent was removed under reduced pressure. The residue was extracted with benzene and organic layer was washed with water and dried.

After removal of benzene, the residue was applied to silica gel column chromatography using benzene as eluent to give 5.9 g of solid product after removal of solvent.

Yield: 5.9 g (98.5%) m.p. 69 — 71°C

ir: 2950, 1690, 1450, 1440, 1421, 1202, 1027, 774, 746 ($cm^{-1}$)

$^1H$—NMR: ($CDCl_3$) 2.58 (3H, S, Me), 3.92 (3H, S, OMe), 7.28 (1H, dd, J=9.6, 9.6, H-5), 7.37 (1H, dd, J=9.6, 9.6, H-7), 7.67 (1H, dd, J=9.6, 9.6, H-6), 8.14 (1H, S, H-2), 8.24 (1H, dd, J=9.6, 1.4, H-4), 9.49 (1H, dd, J=9.6, 1.4, H-8)

(Step 2) 1-methyl azulene (C)

5.91 g of compound B was dissolved in 60 ml of phosphoric acid and the mixture was heated (90—90°C) on a water bath for 15 minutes. After cooling the mixture was poured to 300 ml of ice-water and extracted with n-hexane. The organic layer was separated, washed with water, dried over anhydrous sodium sulfonate and fractionated by silica gel column chromatography using benzene as eluent to give 3.72 g (90.1%) of the desired compound.

ir: 3012, 2930, 1577, 1510, 1455, 1396, 947, 880 ($cm^{-1}$)

$^1H$—NMR: ($CDCl_3$) 2.58 (3H, S, Me), 3.92 (3H, S, OMe), 7.28 (1H, dd, J=9.6, 9.6, H-5), 7.37 (1H, dd, J=9.6, 9.6, H-7), 7.67 (1H, dd, J=9.6, 9.6, H-6), 8.14 (1H, S, H-2), 8.24 (1H, dd, 1=9.6, 1.4, H-4), 9.49 (1H, dd, J=9.6, 1.4, H-8),

(Step 3) Sodium 3-methyl-azulene sulfonate (Compound 1)

Acetic anhydride of 10 ml was added to 1-methyl-azulene of 1.0 g, in cooling condition with ice-water, sulfuric acid of 3.4 g was added dropwise and the reaction mixture was stirred for 4 hours at 0°C. Then, the reaction mixture was made alkaline to pH8—9 by adding 40% of sodium hydroxide solution and the generated solid was collected and dried. The product was recrystallized from 95% ethanol to give 1.1 g of the desired compound.

m.p.: 35—38°C

ir: 1630, 1420, 1220, 750 ($cm^{-1}$)

Compounds 2—34 were obtained by the same sulfonating procedure as in the above-described Step 3 from the corresponding starting materials which are disclosed in the literature (M. Yasunami, Chem. lett., P.579, 1980).

Example 2

Sodium 3-ethyl-azulene sulfonate (Compound 2)

Compound 2 is obtained by using n-butylaldehyde instead of propionaldehyde in Step 1 of Example 1.

m.p.: 28—30°C

ir: 1630, 1452, 1400, 1220, 850 ($cm^{-1}$)

Example 3

Sodium 3-ethyl-7-isopropyl-azulene sulfonate (Compound 3)

Compound 3 was prepared by following the procedures of Example 1, using 5-isopropyl-substituted derivative of oxazulene as a starting material instead of oxazulene (A) and n-butyradldehyde instead of propionaldehyde. The sulfonation is carried out according to the procedure of Step 3 of Example 1.

m.p.: 85—88°C

ir: 1650, 1640, 1564, 1418, 1180, 1050 ($cm^{-1}$)

$^1H$—NMR (DMSO): 1.30 (3H, t, J=J7.4, $CH_3$), 1.33 (6H, d, J=6.6, $(CH_3)_2$—), 3.00 (2H, q, J=7.4, $CH_2$), 3.50 (1H, sept, J=6.6, —C<u>H</u>—$(CH_3)_2$), 4.10 (1H, bs, $H_2O$), 7.20 (1H, d, J=10, H-7), 7.90 (1H, s, H-2), 8.23 (1H, d, J=10, H-4), 9.17 (1H, s, H-8)

Example 4

Sodium 3-ethyl-6-isopropyl-azulene sulfonate (Compound 4)

m.p.: 55—58°C

ir: 1640, 1580, 1410, 1190, 1060 (cm-1)

$^1H$—NMR (DMSO): 1.27 (3H, t, J=7.4, $CH_3$), 1.30 (6H, d, J=6.6, $(CH_3)_2$-), 2.96 (1H, sept, J=6.6, isopropyl), 3.02 (2H, q, J=7.4, $CH_2$), 7.17 (2H, d, J=10, H-5.7), 7.70 (1H, s, H-2), 8.20 (1H, d, J=10, H-8), 8.90 (1H, d, J=10, H-4)

Example 5

Sodium 3-(1′-R, S-1′-5′-dimethyl-4-hexene)-azulene sulfonate (Compound 5)

Compound 5 was prepared according to Example 1, using d.1-citronellal instead of propione aldehyde in Example 1.

m.p.: 78—81°C

ir: 1630, 1560, 1420, 1400, 1190, 1120, 1050, 750 ($cm^{-1}$)

Example 6

Sodium 3-n-butyl-azulene sulfonate (Compound 6)

m.p.: 83—85°C

ir: 1630, 1420, 1400, 1220, 750 ($cm^{-1}$)

Example 7

Sodium 3-methyl-2-ethyl-azulene sulfonate (Compound 7)

Compound 7 was prepared according to Example 1, using 3-(1-pirrolidinyl)-2-pentene instead of propionaldehyde and morpholine in Step 1 of Example 1.

m.p.: 280—283°C

ir: 1640, 1560, 1420, 1190, 1040, 740 ($cm^{-1}$)

Example 8

Sodium 3-(1'-S-1', 5'-dimethyl-4'-hexene)-azulene sulfonate (Compound 8)

Compound 8 was prepared according to Example 1, using d-citronellal instead of proprionaldehyde in Step 1 of Example 1.

m.p.: 78—81°C

ir: 1630, 1560, 1420, 1400, 1190, 1120, 1050, 750 ($cm^{-1}$)

Example 9

Sodium 3-(1'-R-1', 5'-dimethyl-4'-hexene)-azulene sulfonate (Compound 9)

Compound 9 was prepared according to Example 1, using 1-citronellal instead of propionaldehyde in Step 1 of Example 1.

m.p.: 78—81°C

ir: 1630, 1560, 1420, 1400, 1190, 1120, 1050, 750 ($cm^{-1}$)

Example 10

Sodium 3-(1'-S-1', 5'-dimethyl-4'-hexene)-7-isopropyl-azulene sulfonate (Compound 10)

m.p.: 108—110°C

ir: 1630, 1560, 1420, 1220, 1050 ($cm^{-1}$)

Example 11

Sodium 3-(1'-R-1', 5'-dimethyl-4'-hexene)-7-isopropyl-azulene sulfonate (Compound 11)

m.p.: 108—110°C

ir: 1630, 1560, 1420, 1220, 1050 ($cm^{-1}$)

Example 12

Sodium 3-propylazulene sulfonate (Compound 12)

m.p.: 210—215°C (decomp.)

("decomp." stands for decomposition point, hereinafter.)

ir: 3400, 2940, 2850, 1580, 1420, 1400, 1200, 1090, 1020, 960, 880, 750, 740, 670 ($cm^{-1}$)

Example 13

Sodium 3-ethyl-7-isopropyl azulene sulfonate (Compound 13)

m.p.: 91—93°C

ir: 3450, 2950, 1640, 1250, 1070, 1010, 790 ($cm^{-1}$)

Example 14

Sodium 3-n-butylazulene sulfonate (Compound 14)

m.p.: 215—220°C (decomposition point)

ir: 3450, 2900, 1570, 1390, 1190 ($cm^{-1}$)

Example 15

Sodium 3-n-pentyl azulene sulfonate (Compound 15)

m.p.: 217—220°C (decomp.)

ir: 3450, 2900, 1570, 1390, 1190 ($cm^{-1}$)

Example 16

Sodium 5-isopropyl azulene sulfonate (Compound 16)

m.p.: 241—248°C

ir: 3450, 2950, 1640, 1200, 1050 ($cm^{-1}$)

Example 17

Sodium 7-isopropyl-3-n-propyl azulene sulfonate (Compound 17)

m.p.: 138—143°C

ir: 3450, 2950, 1630, 1575, 1470, 1415, 1390, 1220, 1050, 930 ($cm^{-1}$)

Example 18

Sodium 7-isopropyl-3-n-butyl azulene sulfonate (Compound 18)

m.p.: 150—152°C

ir: 3450, 2950, 1640, 1575, 1465 ($cm^{-1}$)

Example 19

Sodium 7-isopropyl-3-n-pentyl azulene sulfonate (Compound 19)

m.p.: 168—170°C

ir: 3400, 2950, 2930, 2860, 1620, 1580, 1470, 1440, 1390, 1190, 1060 ($cm^{-1}$)

Example 20

Sodium 7-isopropyl-3-(1′-R.S.-1′, 5′-dimethyl-4′-hexane) azulene sulfonate (Compound 20)
m.p.: 113—116°C
ir: 3450, 2950, 1640, 1380, 1240, 1180 ($cm^{-1}$)

Example 21

Sodium 7-isopropyl-3-(1′-R-1′, 5′-dimethyl-4′-hexene) azulene sulfonate (Compound 21)
m.p.: 114—117°C
ir: 3450, 2950, 1640, 1380, 1240, 1180 ($cm^{-1}$)

Example 22

Sodium 7-isopropyl-3-(1′-S-1′, 5′-dimethyl-4′-hexene) azulene sulfonate (Compound 22)
m.p.: 115—118°C
ir: 3450, 2950, 1640, 1380, 1240, 1180 ($cm^{-1}$)

Example 23

Sodium 7-isopropyl-3-benzyl azulene sulfonate (Compound 23)
m.p.: 250°C (decomp.)
ir: 3450, 2950, 1640, 1560, 1530, 1460, 1420 ($cm^{-1}$)

Example 24

Sodium 4-methoxy-3-methyl azulene sulfonate (Compound 24)
m.p.: 186—188°C (decomp.)
ir: 3400, 1600, 1570, 1540, 1460, 1370, 1270 ($cm^{-1}$)

Example 25

Sodium 4-methoxy-3-ethyl azulene sulfonate (Compound 25)
m.p.: 60°C
ir: 3400, 2950, 1600, 1570, 1540, 1450, 1370, 1270 ($cm^{-1}$)

Example 26

Sodium 4-methoxy-3-propyl azulene sulfonate (Compound 26)
m.p.: 108—110°C
ir: 3450, 2910, 2850, 1600, 1570, 1530 ($cm^{-1}$)

Example 27

Sodium 4-methoxy-3-butyl azulene sulfonate (Compound 27)
m.p.: 188—190°C (decomp.)
ir: 3450, 2950, 1600, 1570, 1530, 1460 ($cm^{-1}$)

Example 28

Sodium 4-methoxy-3-pentyl azulene sulfonate (Compound 28)
m.p.: 189—192°C (decomp.)
ir: 3450, 2950, 1600, 1560, 1530, 1450 ($cm^{-1}$)

Example 29

Sodium 4-methoxy-3-hexyl azulene sulfonate (Compound 29)
m.p.: 225—228°C (decomp.)
ir: 3450, 2900, 1650, 1560, 1520, 1460 ($cm^{-1}$)

Example 30

Sodium 7-isopropyl-4-methoxy-3-methyl azulene sulfonate (Compound 30)
m.p.: 95—97°C
ir: 3450, 2950, 1650, 1540, 1280, 1180 ($cm^{-1}$)

Example 31

Sodium 7-isopropyl-4-methoxy-3-methyl azulene sulfonate (Compound 31)
m.p.: 108—110°C
ir: 3450, 2950, 1650, 1540, 1260, 1180 ($cm^{-1}$)

Example 32

Sodium 7-isopropyl-4-methoxy-3-propyl azulene sulfonate (Compound 32)
m.p.: 188—190°C
ir: 3450, 2950, 1640, 1560, 1530 ($cm^{-1}$)

Example 33

Sodium 7-isopropyl-4-methoxy-3-butyl azulene sulfonate (Compound 33)

m.p.: 166—168°C

ir: 3450, 2950, 1640, 1560, 1530, 1470 ($cm^{-1}$)

Example 34

Sodium 7-isopropyl-4-methoxy-3-pentyl azulene sulfonate (Compound 34)

m.p.: 123—125°C

ir: 3450, 2950, 1650, 1520, 1260, 1010 ($cm^{-1}$)

Example 35

Sodium-methyl azulene sulfonic acid aluminium salt (Compound 35)

5 g of sodium 3-methyl azulene sulfonate was dissolved in water. To this solution, 2.73 g of AlCl in 10 ml of water after filtration of the residue was added and stirred for 30 minutes at room temperature. The reaction mixture was adjusted to pH 4—4.5 by adding 10% NaOH. The precipitates formed were collected by filtration and washed with water and dried.

m.p.: over 250°C

ir: 3400, 1630, 1580, 1395, 1140, 1040, 740 ($cm^{-1}$)

Al content: 13.96%

The following compounds (Compounds 36—38) were prepared in the same manner as described in Example 35, from the corresponding sodium sulfonate derivatives.

Example 36

3-ethyl azulene sulfonic acid aluminium salt (Compound 36)

m.p.: over 250°C

ir: 3400, 2950, 1580, 1400, 1150, 1050, 750 ($cm^{-1}$)

Al content: 13.00%

Example 37

7-isopropyl-3-methyl azulene sulfonic acid aluminium salt (Compound 37)

m.p.: over 250°C

ir: 3400, 2950, 1420, 1150, 1040 ($cm^{-1}$)

Al content: 9.46%

Example 38

7-isopropyl-3-ethyl azulene sulfonic acid aluminium salt (Compound 38)

m.p.: over 250°C

ir: 3400, 2950, 1580, 1420, 1390, 1150 ($cm^{-1}$)

Al content: 10.58%

## Claims

1. An azulene drrivative of the general formula:

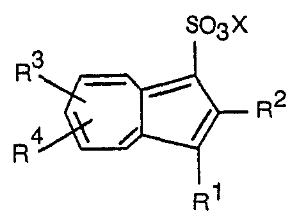

where: $R^1$ is a lower alkyl group, a group of the general formula

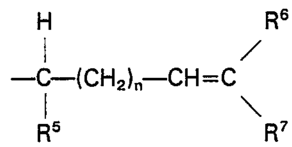

or a benzyl group, $R^5$, $R^6$ and $R^7$ each representing H or a lower alkyl group and n being 1 or 2;

$R^2$ and $R^3$ each represent H or a lower alkyl group;

$R^4$ is H or an alkoxy group;

and X is a Na or $Al(OH)_2$.

2. Sodium 3-ethyl-7-isopropyl-azulene sulfonate.

3. 3-ethyl-7-isopropyl-azulene sulfonic acid aluminium salt.

4. Any of the following compounds:

1) Sodium 3-methyl-azulene sulfonate
2) Sodium 3-ethyl-azulene sulfonate
3) Sodium 3-ethyl-7-isopropyl-azulene sulfonate
4) Sodium 3-ethyl-6-isopropyl-azulene sulfonate
5) Sodium 3-(1'-R, S-1', 5'-dimethyl-4'-hexane) azulene sulfonate
6) Sodium 3-n-butyl-azulene sulfonate
7) Sodium 3-methyl-2-ethyl-azulene sulfonate
8) Sodium 3-(1'-S-1', 5'-dimethyl-4'-hexene)-azulene sulfonate
9) Sodium 3-(1'-R-1', 5'-dimethyl-4'-hexene)-azulene sulfonate
10) Sodium 3-(1'-S-1', 5'-dimethyl-4'-hexene)-7-isopropyl-azulene sulfonate
11) Sodium 3-(1'-R-1', 5'-dimethyl-4'-hexene)-7-isopropyl-azulene sulfonate
12) Sodium 3-propyl-azulene sulfonate
13) Sodium 3-methyl-7-isopropyl-azulene sulfonate
14) Sodium 3-n-butyl-azulene sulfonate
15) Sodium 3-n-pentyl-azulene sulfonate
16) Sodium 5-isopropyl-azulene sulfonate
17) Sodium 7-isopropyl-3-n-propyl-azulene sulfonate
18) Sodium 7-isopropyl-3-n-butyl-azulene sulfonate
19) Sodium 7-isopropyl-3-n-pentyl-azulene sulfonate
20) Sodium 7-isopropyl-3-(1'-R, S-1', 5'-dimethyl-4'-hexene)-azulene sulfonate
21) Sodium 7-isopropyl-3-(1'-R-1', 5'-dimethyl-4'-hexene)-azulene sulfonate
22) Sodium 7-isopropyl-3-(1'-S-1', 5'-dimethyl-4'-hexene)-azulene sulfonate
23) Sodium 7-isopropyl-3-benzyl-azulene sulfonate
24) Sodium 4-methoxy-3-methyl-azulene sulfonate
25) Sodium 4-methoxy-3-ethyl-azulene sulfonate
26) Sodium 4-methoxy-3-propyl-azulene sulfonate
27) Sodium 4-methoxy-3-butyl-azulene sulfonate
28) Sodium 4-methoxy-3-pentyl-azulene sulfonate
29) Sodium 4-methoxy-3-hexyl-azulene sulfonate
30) Sodium 7-isopropyl-4-methoxy-3-methyl-azulene sulfonate
31) Sodium 7-isopropyl-4-methoxy-3-ethyl-azulene sulfonate
32) Sodium 7-isopropyl-4-methoxy-3-propyl-azulene sulfonate
33) Sodium 7-isopropyl-4-methoxy-3-butyl-azulene sulfonate
34) Sodium 7-isopropyl-4-methoxy-3-pentyl-azulene sulfonate
35) 3-methyl-azulene sulfonic acid aluminium salt
36) 3-ethyl-azulene sulfonic acid aluminium salt
37) 7-isopropyl-3-methyl-azulene sulfonic acid aluminium salt
38) 7-isopropyl-3-ethyl-azulene sulfonic acid aluminium salt

5. A compound according to any preceding claim, for use in therapeutic treatment of the human or animal body.

6. A compound according to claim 5, wherein said treatment is to prevent or treat ulcer or inflammation.

7. A therapeutic composition comprising a pharmaceutically acceptable carrier and, as an active ingredient, a compound according to any of claims 1 to 6.

8. A therapeutic composition according to claim 7, wherein the active ingredient is present in an amount effective to treat peptic ulcer.

9. A therapeutic composition according to claim 7, wherein the active ingredient is present in an amount effective to treat inflammation.

10. A method of preparing a compound according to claim 1, comprising:
reacting a compound of the formula:

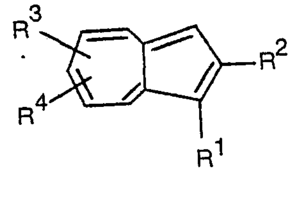

where $R^1$, $R^2$, $R^3$ and $R^4$ are as defined in claim 1 with a sulfonating agent and converting the resulting sulfonic acid to its sodium or aluminium salt.

11. A method according to claim 10 wherein the sulfonating agent is sulfonic acid or sulfonic anhydride-pyridine complex.

12. A method according to claim 10 or 11, wherein said resulting sulfonic acid is converted to is sodium salt and the sodium salt is reacted with an aluminium salt in solution to yield an aluminium salt according to claim 1.

13. A method according to claim 12, wherein the aluminium salt is an aqueous solution of $Al(OH)_3$ or $AlCl_3$.

14. An azulene derivative of the general formula given in claim 1 where $R^1$, $R^2$, $R^3$ and $R^4$ are as defined in claim 1 but X is H.

15. A method of preparing a therapeutic composition comprising admixing a pharmaceutically acceptable carrier and a compound according to any of claims 1 to 6.

16. A method of preparing a compound according to claim 14, comprising reacting a compound of the formula

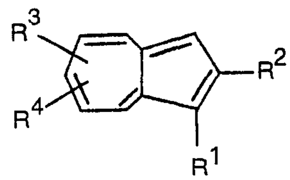

where $R^1$, $R^2$, $R^3$ and $R^4$ are as defined in claim 1 with a sulfonating agent.

**Patentansprüche**

1. Ein Azulenderivat der allgemeinen Formel:

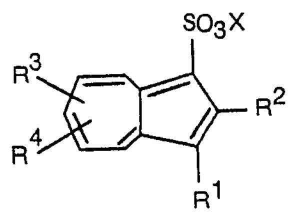

in welcher $R^1$ eine niedere Alkylgruppe, eine Gruppe der allgemeinen Formel

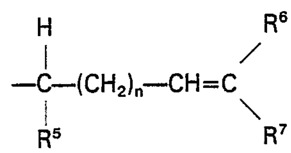

oder eine Benzylgruppe ist, wobei $R^5$, $R^6$ und $R^7$ jedes H oder eine niedere Alkylgruppe darstellt und n 1 oder 2 bedeutet;
$R^2$ und $R^3$ jedes H oder eine niedere Alkylgruppe darstellt;
$R^4$ H oder eine Alkoxygruppe bedeutet, und
X Na oder Al $(OH)_2$ ist.

2. Natrium-3-äthyl-7-isopropyl-azulensulfonat.

3. 3-Aethyl-7-isopropyl-azulensulfonsäure-Aluminiumsalz.

4. Jede der folgenden Verbindungen:
1) Natrium-3-methyl-azulensulfonat,
2) Natrium-3-äthyl-azulensulfonat,
3) Natrium-3-äthyl-7-isopropyl-azulensulfonat,
4) Natrium-3-äthyl-6-isopropyl-azulensulfonat,
5) Natrium-3-(1'-R, S-1', 5'-dimethyl-4'-hexan)-azulensulfonat,
6) Natrium-3-n-butyl-azulensulfonat,
7) Natrium-3-methyl-2-äthyl-azulensulfonat,
8) Natrium-3-(1'-S-1', 5'-dimethyl-4'-hexen)-azulensulfonat,
9) Natrium-3-(1'-R-1', 5'-dimethyl-4'-hexen)-azulensulfonat,
10) Natrium-3-(1'-5-1', 5'dimethyl-4'-hexen)-7-isopropyl-azulensulfonat,
11) Natrium-3-(1'-R-1', 5'-dimethyl-4'hexen)-7-isopropyl-azulensulfonat,
12) Natrium-3-propyl-azulensulfonat,
13) Natrium-3-methyl-7-isopropyl-azulensulfonat,
14) Natrium-3-n-butyl-azulensulfonat,
15) Natrium-3-n-pentyl-azulensulfonat,

16) Natrium-5-isopropyl-azulensulfonat,
17) Natrium-7-isopropyl-3-n-propyl-azulensulfonat,
18) Natrium-7-isopropyl-3-n-butyl-azulensulfonat,
19) Natrium-7-isopropyl-3-n-pentyl-azulensulfonat,
20) Natrium-7-isopropyl-3-(1'-R, S-1', 5'-dimethyl-4'-hexen)-azulensulfonat,
21) Natrium-7-isopropyl-3-(1'-R-1', 5'-dimethyl-4'-hexen)-azulensulfonat,
22) Natrium-7-isopropyl-3-(1'-S-1', 5'-dimethyl-4'-hexen)-azulensulfonat,
23) Natrium-7-isopropyl-3-benzyl-azulensulfonat,
24) Natrium-4-methoxy-3-methyl-azulensulfonat,
25) Natrium-4-methoxy-3-äthyl-azulensulfonat,
26) Natrium-4-methoxy-3-propyl-azulensulfonat,
27) Natrium-4-methoxy-3-butyl-azulensulfonat,
28) Natrium-4-methoxy-3-pentyl-azulensulfonat,
29) Natrium-4-methoxy-3-hexyl-azulensulfonat,
30) Natrium-7-isopropyl-4-methoxy-3-methyl-azulensulfonat,
31) Natrium-7-isopropyl-4-methoxy-3-äthyl-azulensulfonat,
32) Natrium-7-isopropyl-4-methoxy-3-propyl-azulensulfonat,
33) Natrium-7-isopropyl-4-methoxy-3-butyl-azulensulfonat,
34) Natrium-7-isopropyl-4-methoxy-3-pentyl-azulensulfonat,
35) 3-Methyl-azulen-sulfonsäuren-Aluminiumsalz,
36) 3-Aethyl-azulen-sulfonsäure-Aluminiumsalz,
37) 7-Isopropyl-3-methyl-azulen-sulfonsäure-Aluminiumsalz,
38) 7-Isopropyl-3-äthyl-azulen-sulfonsäure-Aluminiumsalz.

5. Eine Verbindung nach einem der vorstehenden Ansprüche zur Verwendung bei der therapeutischen Behandlung des menschlichen oder tierischen Körpers.

6. Eine Verbindung nach Anspruch 5, dadurch gekennzeichnet, dass diese Behandlung zur Verhütung oder Behandlung von Geschwüren oder Enzündungen dient.

7. Eine therapeutische Zusammensetzung, welche einen pharmazeutisch annehmbaren Träger und als aktiven Bestandteil eine Verbindung nach einem der Ansprüche 1 bis 6 enthält.

8. Eine therapeutische Zusammensetzung nach Anspruch 7, dadurch gekennzeichnet, dass der aktive Bestandteil in einer zur Behandlung peptidischer Geschwüre wirksamen Menge vorhanden ist.

9. Eine therapeutische Zusammensetzung nach Anspruch 7, dadurch gekennzeichnet, dass der aktive Bestandteil in einer zur Behandlung von Entzündungen wirksamen Menge vorhanden ist.

10. Ein Verfahren zur Herstellung einer Verbindung nach Anspruch 1, dadurch gekennzeichnet, dass eine Verbindung der Formel:

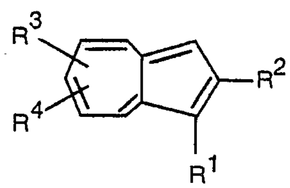

in welcher $R^1$, $R^2$, $R^3$ und $R^4$ dieselbe Bedeutung wie in Anspruch 1 aufweisen, mit einem Sulfonierungsmittel behandelt und die erhaltene Sulfonsäure in ihr Natrium- oder Aluminiumsalz umgewandelt wird.

11. Ein Verfahren nach Anspruch 10, dadurch gekennzeichnet, dass das Sulfonierungsmittel Sulfonsäure oder der Sulfonsäureanhydrid-Pyridin-Komplex ist.

12. Ein Verfahren nach Anspruch 10 oder 11, dadurch gekennzeichnet, dass die erhaltene Sulfonsäure in das Natriumsalz umgewandelt wird und das Natriumsalz mit einem Aluminiumsalz in Lösung umgesetzt wird, um ein Aluminiumsalz nach Anspruch 1 zu ergeben.

13. Ein Verfahren nach Anspruch 12, dadurch gekennzeichnet, dass das Aluminiumsalz eine wässerige Lösung von $Al(CO)_3$ oder $AlCl_3$ ist.

14. Ein Azulenderivat der allgemeinen Formel nach Anspruch 1, in welcher $R^1$, $R^2$, $R^3$ und $R^4$ die in Anspruch 1 gegebene Bedeutung aufweisen, aber X Wasserstoff bedeutet.

15. Ein Verfahren zur Herstellung einer therapeutischen Zusammensetzung, dadurch gekennzeichnet, dass ein pharmazeutisch annehmbares Salz und eine Verbindung gemäss einem der Ansprüche 1 bis 6 miteinander vermischt werden.

16. Ein Verfahren zur Herstellung einer Verbindung nach Anspruch 14, dadurch gekennzeichnet, dass eine Verbindung der Formel:

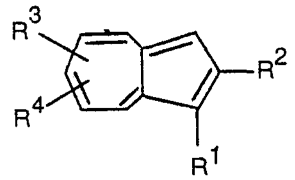

in welcher $R^1$, $R^2$, $R^3$ und $R^4$ dieselbe Bedeutung wie in Anspruch 1 aufweisen, mit einem Sulfonierungsmittel umgesetzt wird.

**Revendications**

1. Dérivé d'azulène répondant à la formule générale suivante:

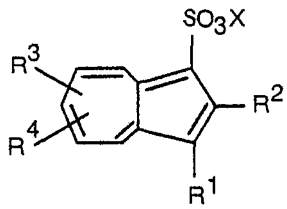

où $R^1$ représente un groupe alkyle inférieur, un groupe à formule générale

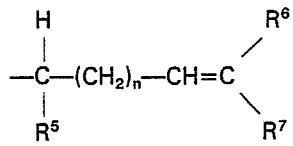

ou un groupe benzyle, où $R^5$, $R^6$ et $R^7$ représentent chacun H ou un groupe alkyle inférieur et n=1 ou 2;
$R^2$ et $R^3$ représentent chacun H ou un groupe alkyle inférieur;
$R^4$ est H ou un groupe alkoxy; et
X est Na ou Al $(OH)_2$.

2. 3-Ethyl-7-isopropyl-azulène sulfonate sodique.
3. Sel d'aluminium d'acide 3-éthyl-7-isopropyl-azulène-sulfonique.
4. Chacun des composés suivants:

1) 3-méthyl-azulènepsulfonate sodique
2) 3-éthyl-azulènepsulfonate sodique
3) 3-éthyl-7-isopropyl-azulène-sulfonate sodique
4) 3-éthyl-6-isopropyl-azulènepsulfonate sodique
5) 3-(1'-R, S-1', 5'-diméthyl-4'-hexane)-azulène-sulfonate sodique
6) 3-n-butyl-azulène-sulfonate sodique
7) 3-méthyl-2-éthyl-azulène-sulfonate sodique
8) 3-(1'-S-1', 5'-diméthyl-4'-hexène)-azulène-sulfonate sodique
9) 3-(1'-R-1', 5'-diméthyl-4'-hexène)-azulène-sulfonate sodique
10) 3-(1'-S-1', 5'-diméthyl-4'-hexène)-7-isopropyl-azulène-sulfonate sodique
11) 3-(1'-R-1', 5'-diméthyl-4'-hexène)-7-isopropyl-azulène-sulfonate sodique
12) 3-propyl-azulène-sulfonate sodique
13) 3-méthyl-7-isopropyl-azulène-sulfonate sodique
14) 3-n-butyl-azulène-sulfonate sodique
15) 3-n-pentyl-azulène-sulfonate sodique
16) 5-isopropyl-azulène-sulfonate sodique
17) 7-isopropyl-3-n-propyl-azulène-sulfonate sodique
18) 7-isopropyl-3-n-butyl-azulène-sulfonate sodique
19) 7-isopropyl-3-n-pentyl-azulène-sulfonate sodique
20) 7-isopropyl-3-(1'-R, S-1', 5'-diméthyl-4' -hexène)-azulène-sulfonate sodique
21) 7-isopropyl-3-(1'-R-1', 5'-diméthyl-4' -hexène)-azulène-sulfonate sodique
22) 7-isopropyl-3-(1'-S-1', 5'-diméthyl-4'- hexène)-azulène-sulfonate sodique
23) 7-isopropyl-3-benzyl-azulène-sulfonate sodique
24) 4-méthoxy-3-méthyl-azulène-sulfonate sodique
25) 4-méthoxy-3-éthyl-azulène-sulfonate sodique
26) 4-méthoxy-3-propyl-azulène-sulfonate sodique
27) 4-méthoxy-3-butyl-azulène-sulfonate sodique

28) 4-méthoxy-3-pentyl-azulène-sulfonate sodique
29) 4-méthoxy-3-hexyl-azulène-sulfonate sodique
30) 7-isopropyl-4-méthoxy-3-méthyl-azulène-sulfonate sodique
31) 7-isopropyl-4-méthoxy-3-éthyl-azulène-sulfonate sodique
32) 7-isopropyl-4-méthoxy-3-propyl-azulène-sulfonate sodique
33) 7-isopropyl-4-méthoxy-3-butyl-azulène-sulfonate sodique
34) 7-isopropyl-4-méthoxy-3-pentyl-azulène-sulfonate sodique
35) sel d'aluminium d-acide 3-méthyl-azulène-sulfonique
36) sel d'aluminium d'acide 3-éthyl-azulène-sulfonique
37) sel d'aluminium d'acide 7-isopropyl-3-méthyl-azulène-sulfonique
38) sel d'aluminium d'acide 7-isopropyl-3-éthyl-azulène-sulfonique

5. Composé selon l'une ou l'autre des revendications précédentes, destiné à l'emploi dans le traitement thérapeutique de l'organisme humain ou animal.

6. Composé selon la revendication 5, dans lequel ledit traitement thérapeutique est un traitement préventif ou curatif anti-ulcéreux ou anti-inflammatoire.

7. Préparation thérapeutique contenant un véhicule acceptable au point de vue pharmaceutique et, en guise d'un ingrédient actif un composé selon l'une ou l'autre des revendications 1 à 6.

8. Préparation thérapeutique selon la revendication 7, dans laquelle 1'ingrédient actif est présent en une quantité appropriée au traitement de l'ulcère peptique.

9. Préparation thérapeutique selon la revendication 7, dans lequel l'ingrédient actif est présent en une quantité appropriée au traitement anti-inflammatoire.

10. Procédé de préparation d'un composé selon la revendication 1, comportant la mise en réaction d'un composé selon la formule générale:

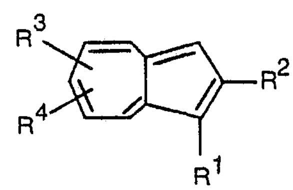

où $R^1$, $R^2$, $R^3$ et $R^4$ ont la signification définie dans la revendication 1, avec un agent de sulfonation, suivie de transformation de l'acide sulfonique ainsi formé en son sel de sodium ou d'aluminium.

11. Procédé selon la revendication 10, dans lequel l'agent de sulfonation est l'acide sulfonique ou un complexe d'anhydride sulfonique et de pyridine.

12. Procédé selon la revendication 10 ou 11, dans lequel l'acide sulfonique ainsi formé est transformé en son sel sodique et le sel sodique ainsi obtenu est, par réaction avec un sel d'aluminium en solution, transformé en un sel d'aluminium selon la revendication 1.

13. Procédé selon la revendication 12, dans lequel le sel d'aluminium est une solution aqueuse de Al $(OH)_3$ ou de $AlCl_3$.

14. Dérivé d'azulène répondant à la formule générale donnée dans la revendication 1 où $R^1$, $R^2$, $R^3$ et $R^4$ ont la signification définie dans la revendication 1 mais où est H.

15. procédé de préparation d'un composé thérapeutique comportant le mélange d'un véhicule acceptable au point de vue pharmaceutique et un composé selon l'une ou l'autre des revendications 1 à 6.

16. Procédé de préparation d'un composé selon la revendication 14, comportant la mise en réaction d'un composé selon la formule:

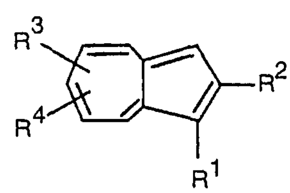

où $R^1$, $R^2$, $R^3$ et $R^4$ ont la signification définie dans la revendication 1 avec un agent de sulfonation.